Europäisches Patentamt

(19) European Patent Office

Office européen des brevets

(11) Publication number: **0 322 591**
A2

(12) # EUROPEAN PATENT APPLICATION

(21) Application number: 88120051.3

(51) Int. Cl.⁴: **C12Q 1/04 , C12Q 1/18**

(22) Date of filing: **01.12.88**

(30) Priority: **01.12.87 US 126654**

(43) Date of publication of application:
**05.07.89 Bulletin 89/27**

(84) Designated Contracting States:
**AT BE CH DE ES FR GB IT LI NL**

(71) Applicant: **ABBOTT LABORATORIES**

**Abbott Park Illinois 60064(US)**

(72) Inventor: **Elmore, John J., Jr.**
**37874 DeWoody Road**
**Waukegan Illinois 60087(US)**
Inventor: **Kimberlin, Cecilia**
**703 Dawes Street**
**Libertyville Illinois 60048(US)**
Inventor: **Ness, David A.**
**2111 Lorraine Avenue, No. 2N**
**Waukegan Illinois 60087(US)**

(74) Representative: **Modiano, Guido et al**
**MODIANO, JOSIF, PISANTY & STAUB**
**Modiano & Associati Baaderstrasse 3**
**D-8000 München 5(DE)**

(54) **Antibiotic resistance testing assay.**

(57) The present invention provides methods and compositions for the detection of bacteria in specimen samples as well as for determinations of antibiotic resistance. The method involves the addition of a test sample to a nutrient medium containing an antibiotic which may be capable of interfering with the metabolism of the microorganism. The medium is then incubated. An indicator such as a tetrazolium compound which will produce a color change in the presence of a microorganism and an electron carrier such as phenazine methosulfate are added to the medium immediately following the incubation period. The specimen is graded according to any color change produced by an antibiotic-resistant microorganism present in the test sample. The invention may also be used without the addition of an antibiotic as a means for determining whether a microorganism is present in a test sample.

# ANTIBIOTIC RESISTANCE TESTING ASSAY

## BACKGROUND OF THE INVENTION

### Technical Field

This invention relates generally to the fields of methods, compositions, and products for detecting microorganisms. More particularly, the present invention relates to a novel method for rapidly determining antibiotic sensitivity.

### Background

A variety of assays have been developed to detect the presence of bacteria and to determine the bacterium's sensitivity to antibiotics. It is desirable to achieve each of these objectives as rapidly as possible for the effective diagnosis of infectious disease and the initiation of treatment. Urinary tract infections are among the most common of bacterial diseases and mostly result from ascending infection by microorganisms introduced through the urethra. Under normal conditions, urine is sterile, but contamination from vaginal, urethral, or perineal flora may contribute up to 1,000 ($10^3$) organisms per milliliter in properly collected specimens. A bacterial count of 100,000 ($10^5$) or more organisms per milliliter of urine is considered significant bacteriuria.

Significant bacteriuria may arise due to microbial invasion of any of the tissues of the urinary tract, or may result from simple bacterial multiplication without tissue invasion. In the latter case, the infection may be asymptomatic, yet early treatment could prevent the development of a more serious condition. Therefore, the rapid detection of low concentrations of bacteria would facilitate an early and specific diagnosis, and a determination of antibiotic resistance would indicate a likely therapy. Furthermore, repeated tests during therapy may be necessary to insure that a prescribed antibiotic is in fact effective in treating an infection.

A major problem encountered with conventional bacterial detection and sensitivity tests is the length of time that passes between obtaining a specimen to determining the microorganism's presence and selecting a suitable antimicrobial agent for treatment. Laboratory methods based on culturing microorganisms, e.g., the calibrated loop direct streak method, require incubation periods ranging from 18 to 24 hours before results can be determined. These prolonged periods are necessary to allow sufficient bacterial growth such that colony formation or turbidity may be detected by visual observation. If the culture is grown on a plate, as in the agar diffusion test, a disk impregnated with antibiotic can be placed on the medium to detect the microorganism's antibiotic sensitivity or antibiotic resistance. When the antibiotic contacts an antiobiotic-sensitive microorganism the microorganism's growth is inhibited thus creating an area or zone that is free of bacterial growth. As the antibiotic diffuses through the medium its concentration decreases at the periphery of the inhibition zone and will become too low to further inhibit growth. As a result, colony formation appears at the border of the inhibition zone. Prolonged incubation periods are necessary to allow the border between the growing bacteria and the antibiotic inhibition zone to become visible to the naked eye. These laboratory methods are time consuming to perform and require considerable clinical training and facilities.

Other conventional assay methods have used pH changes to detect microbial strains. These assays are based on the microorganism's production of by-products, such as acidic or alkaline by-products. A pH change may be measured by a pH meter, or an indicator such as a pH sensitive dye compound may be added to the medium so that a color change is generated as the pH changes. Assay methods based on the detection of pH changes may require about 4 to 18 hours of culture time for the microorganism to generate a sufficient amount of acid to react with the indicator or be measurable with a pH meter. Although faster than the laboratory culture method, the utility of such assays is still limited because they are inapplicable to the numerous strains of microorganisms that are able to metabolize compounds with no resultant production of acid or other cause for a change in pH.

## SUMMARY OF THE INVENTION

2

The present invention provides a method for determining the antibiotic resistance of microorganisms in a test sample, such as a urine specimen. In a preferred embodiment, a test sample suspected of containing a microorganism is added to a nutrient medium containing an antibiotic which may be capable of interfering with the metabolism of the microrganism. The medium is incubated for about three hours. Immediately following the incubation an indicator, capable of undergoing a color change in the presence of a microorganism without substantially interfering with the metabolism of the microorganism, and an electron carrier, capable of facilitating the production of a color change of said indicator by the microorganism, are added to the medium. Shortly after adding the indicator/electron carrier, usually about fifteen minutes, the specimen is graded according to any color change produced by an antibiotic- resistant microorganism present in the test sample. The invention may also be used without the addition of an antibiotic if the assay is used only as a means of determining whether a microorganism is present in a test sample.

In a preferred embodiment of the invention the indicator is a tetrazolium compound. The compound 3-(4,5-dimethylthiazol-2-yl)-2,5-diphenyl-2H-tetrazolium bromide is especially preferred. The preferred electron carriers are N-methylphenazonium methosulphate (PMS), N-ethylphenazonium ethosulfate, and phenazine N-oxide, with PSM being the especially preferred electron carrier.

Because of the use of the indicator and the electron carrier compounds, a very small amount of test sample may be used even though the sample contains a low concentration of microorganisms. Furthermore, the results of the color developing reaction are observable in a fraction of the time needed to produce visible bacterial colonies or detectable pH changes in alternative assays.

The present invention also encompasses devices or kits with which the assays may be performed.

## DETAILED DESCRIPTION OF THE INVENTION

The present invention relates to the detection of bacteria in specimen samples as well as to the determination of antibiotic resistance. The invention may be used to assay any specimen suspected of being bacteria-contaminated ( e.g., food, tissue, water, etc. with appropriate emulsification of solids in a buffer solution). The invention, however, is particularly useful in assays of biological fluids such as urine, spinal fluid, and blood, or suspensions of human tissue. The preferred biological fluid for use in the present invention is urine.

The invention represents a novel approach to determining the resistance of uropathogens to appropriate antimicrobial agents in that the sample (urine) is tested directly rather than using culture methods, i.e., growing and isolating the uropathogens first. The time for testing and obtaining results is shortened from the routine culture and sensitivity test methods. And, the lack of extended culture periods results in less interference by the external contaminants which do not have time to multiply and falsely indicate significant bacteriuria.

The present invention provides a novel and rapid method for determining the presence and antibiotic sensitivity of microorganisms in a test sample. The reagents for the process include a nutrient, antibiotic(s), an indicator compound, an electron carrier, and a detergent.

According to a preferred embodiment of the test method, Mueller-Hinton broth is used as the basic nutrient medium as recommended by the National Committee for Clinical Lab Standards (NCCLS). The broth is supplemented with dextrose to enhance the growth or multiplication of certain microorganisms and thereby further enhance the speed of the assay.

The indicator compound is a dye which enables a chromogenic determination of the presence of bacteria, and when the dye is used with an antibiotic, it enables a chromogenic determination of the bacterium's antibiotic sensitivity or resistance. Preferably, the indicator is a tetrazolium dye which is relatively colorless in an unreduced state but which is converted to a color when reduced to its constituent formazan compounds by a microorganism. Of particular importance to the invention is the choice of the tetrazolium compound because the tetrazolium dye itself may be inhibitory to the growth of certain microorganisms. The present invention, however, enables the use of a tetrazolium dye directly in the culture medium at a concentration that minimizes inhibitory effects while maintaining a concentration sufficient to produce a visually detectable color change.

Preferred tetrazolium dyes useful in the present invention include 2,3,5-triphenyl-2H-tetrazolium chloride (Red tetrazolium; Polysciences, Inc., Warrington, PA); 2,5-diphenyl-3-[alpha-naphthyl]-1-tetrazolium chloride (Tetrazolium violet; Sigma Chemical Co., St.Louis, MO); 3-(4,5-dimethylthiazol-2-yl)-2,5-diphenyl-2H-

3

tetrazolium bromide (MTT; Sigma or Polysciences); 2-(4-iodophenyl)-3-(4-nitrophenyl)-5-phenyl tetrazolium chloride (INT; Polysciences); 3,3'-(4,4'-biphenylene)bis(2,5-diphenyl-2H-tetrazolium chloride) (Neotetrazolium; Sigma); 3,3'-(3,3-dimethoxy-4,4'-biphenylene)bis(2,5-diphenyl-2H-tetrazolium chloride) (Blue tetrazolium; Polysciences); 3,3'-(3,3'-dimethoxy-4,4'-biphenylene)bis[2-(4-nitrophenyl)-5-phenyl-2H-tetrazolium chloride] (Nitroblue tetrazolium; NBT; Polysciences); and 3,3'-(3,3'-dimethoxy-4,4'-biphenylene)-bis[2,5-bis(4-nitrophenyl)-2H-tetrazolium chloride] (Tetranitroblue tetrazolium; TNBT; Sigma). The most preferred tetrazolium compounds are MTT, INT, and nitroblue tetrazolium, all of which exhibit good color (formazan) production within 15 to 30 minutes of their addition to metabolically active bacteria. An especially preferred tetrazolium compound for the present invention is MTT. MTT is chosen for its minimal affect on bacterial growth as well as its distinct purple color change and generally soluble formazan (the INT formazan is red, thus conflict with some urines; and the NBT formazan which is dark blue is generally insoluble). In addition, the present invention allows the performance of the test using small sample sizes so that the formation of the dye's formazan constituents are not affected. Furthermore, the other test reagents are chosen for their inability to break down the tetrazolium compound, thus offering a reduction or an elimination of non-specific background color formation which can otherwise interfere with the test signal.

An electron carrier compound is used to facilitate the microorganism's reduction of tetrazolium by transporting electrons for use in the redox reaction. The electron carrier compounds include N-methylphenazonium methosulphate (Phenazine methosulphate; PMS; Polysciences); N-ethylphenazonium ethosulfate (Phenazine ethosulfate; PES; ICN Biochemicals, Plainview, NY); and phenazine N-oxide (PNO; ICN Biochemicals). In a preferred embodiment of the invention, phenazine methosulfate is used as the electron carrier because the MTT/PMS combination showed the best sensitivity and kinetics of color development in detecting bacterial growth. The electron carrier is not essential to produce the color change, but it enhances the speed of the procedure by substantially increasing the speed of the redox reaction as well as the intensity of the color change.

Certain nonionic detergents can be added to the medium to make it easier for the electron carrier to reach the site of the redox reaction in the microorganism. Preferred detergents which have been found include polyethyleneglycol para-isooctyl-phenyl ether (Triton X-100, Pierce Chemical Co., Rockford, IL); Tergitol 15-S-15 (Sigma Chemical Co., St. Louis, MO); Tergitol 15-S-30 (Sigma); NP-35 (Sigma); Nonidet-P40 (Sigma); polyethoxyethanol lauryl ether (BRIJ-35, Sigma); polyoxyethanol sorbitan monolaurate (Tween-20, Fischer Scientific, Orangeburg, NJ); and Tween-80 (Difco Laboratories, Detroit, MI). An especially preferred nonionic detergent for the present invention is Tween-80.

When the present invention is used to determine the antibiotic sensitivity of a microorganism, an antibiotic is added to the culture medium which is then inoculated with the microorganism. If the microorganism is resistant to the antibiotic then the microorganism's growth is not inhibited, and the tetrazolium compound will be reduced in the presence of the electron carrier. As the tetrazolium undergoes reduction, it is converted to the formazan and a color change occurs in the culture medium. Alternatively, if the microorganism is sensitive to the antibiotic, then the microorganism's growth is inhibited and the tetrazolium is not reduced or is reduced in an amount too low to produce the characteristic color change.

The antibiotics used in the present invention, either singly or as a panel, are selected from ampicillin, sulfamethoxazole, trimethoprim, sulfamethoxazole/trimethoprim, cephalothin, nalidixic acid, nitrofurantoin, tetracycline, and norfloxacin. These antibiotics were selected because they represent those that are commonly used for the treatment of urinary tract infections in non-hospitalized patients, i.e., they are effective against the most common uropathogens including *Escherichia coli*, *Proteus*, *Klebsiella*, *Enterobacter*, *Staphlococcus*, and *Streptococcus* strains. It will be appreciated by those skilled in the art that other antibiotics can be used in the present invention with the choice of antibiotic being related to the bacterial contamination suspected of being in a test sample.

Another embodiment of the present invention entails the use of the reagents, except the antibiotic, to generate a color change to detect the presence of a microorganism without concern for the microorganism's antibiotic sensitivity.

Because of the use of small amounts of test sample having low concentrations of microorganisms, and the use of the MTT and the electron carrier compounds, the results of the redox reaction are observable in a fraction of the time needed to produce visible bacterial colonies or detectable pH changes in alternative assays. The present invention offer 1) the unambiguous detection of the microorganism's metabolic activity, 2) a more rapid detection of metabolism to provide a rapid method for detecting the presence of microorganisms or for antibiotic sensitivity testing, and 3) the ability to directly test a clinical sample such as a urine sample.

The present invention is further illustrated by the following examples.

Examples

The examples concern rapid bacterial detection and antibiotic sensitivity testing procedures. The following examples are representative of the methods for preparing the preferred reagents and test samples for use in the present invention. The bacteria used in the test panel represent those most commonly found in urinary tract infections in non-hospitalized patients. The invention will also be effective for testing other microbes although the length of incubation and ranges of reagents must be modified to be consistent with organisms with slower metabolic activity.

Although urine is normally considered a sterile body fluid, it may become contaminated with flora from external genitalia. To avoid such contamination and its affects on the accuracy of an assay, the first voided morning specimen should be collected whenever possible. If this is not possible, then the urine should be held in the bladder as long as possible to enhance the number of bacteria per milliliter of urine. A procedure for collection of a "clean-catch" midstream urine should then be followed. Briefly, the urethral area is washed with mild soap and water, then rinsed. A small amount of urine is then voided into the toilet, and a portion of the remaining urine is voided into a sterile specimen collection container. Once collected, the specimen should be tested as soon as possible. Urine should be tested within two hours after collection, or it should be refrigerated (at 4° C) and tested within 24 hours.

EXAMPLE 1: Preparation of the nutrient medium

Mueller-Hinton broth was used as the basic nutrient medium. The broth was supplemented with dextrose to enhance the growth of the microorganisms. Dehydrated Mueller-Hinton broth (21 g, Difco Laboratories, Detroit, MI) was rehydrated by suspension in distilled or deionized water (one liter) with gentle warming. The medium was sterilized by autoclaving (15 minutes at 15 lbs pressure, 121° C) or by sterile filtration. A sterile 10% dextrose solution was added (ten milliliters) to make a 0.1% dextrose-supplemented medium. Before use the nutrient broth was filter sterilized by passing it through a 0.22 micron filter.

EXAMPLE 2: Preparation of inocula for testing - clinical isolates and clinical urines

Clinical isolates and the following American Type Culture Collection (ATCC) strains were used after a 16 to 24 hour subculture incubation: *Enterobacter aerogenes, Enterobacter cloacae, Escherichia coli, Klebsiella pneumoniae, Proteus mirabilis, Proteus vulgaris, Staphlococcus epidermidis, Staphlococcus saprophyticus, Staphlococcus aureus, Streptococcus faecalis, Streptococcus faecium,* and *Streptococcus agalactiae.* A well-isolated colony was removed from each subculture and transferred to a tube of dextrose-supplemented Mueller-Hinton broth (three milliliters). The tubes were vortexed and then incubated at 37° C until the turbidity reached approximately an absorbence of 0.54 at 630 nm, in three to five hours. A test standard of *Escherichia coli (E. coli*; ATCC strain #25922)was prepared in dextrose-supplemented Mueller-Hinton broth, to be used as a procedural control culture, and the turbidity was adjusted to approximately an absorbence of 0.54 at 630 nm, representing approximately $10^8$ bacterial cells per milliliter. The absorbence readings were recorded.

The test standard and a spectrophotometer were used to adjust all bacterial suspensions to be tested to approximately the same turbidity. The adjustments were made by adding dextrose-supplemented Mueller-Hinton broth. Then all suspensions were further diluted 1:5 with dextrose-supplemented Mueller-Hinton broth. Preferably, the urine samples suspected of containing microorganisms were midstream urines, freshly collected (within one hour of testing). If not fresh, the urine samples were prewarmed at 37° C for thirty minutes to two hours.

EXAMPLE 3: Preparation of antibiotic stock solutions

The antibiotic stock solutions were prepared according to NCCLS guidelines (Volume 5, No. 22). The weights given in Table 1 are based on ten milliliter amounts of stock solution, and volume adjustments were made if the actual antibiotic weight was different from that given in Table 1.

5

TABLE 1

| ANTIBIOTIC | WEIGHT (mg) | CONCENTRATION($\mu$G/ml) |
|---|---|---|
| ampicillin | 5.12 | 512 |
| sulfamethoxazole | 51.20 | 5120 |
| trimethoprim | 5.12 | 512 |
| cephalothin | 5.12 | 512 |
| nalidixic acid | 5.12 | 512 |
| nitrofurantoin | 10.00 | 1000 |
| tetracycline | 5.12 | 512 |
| norfloxacin | 4.00 | 400 |

The antibiotic stock solutions were then prepared according to Table 2.

TABLE 2

| ANTIBIOTIC | SOLVENT | DILUENT |
|---|---|---|
| ampicillin | phosphate buffer, pH 6.0, 0.1M | phosphate buffer, pH 8.0, 0.1M |
| sulfamethoxazole | 1/2 volume hot water, minimal 2.5 N NaOH | water |
| trimethoprim | 0.05 N HCl, 10% of final volume | water |
| cephalothin | phosphate buffer, pH 6.0, 0.1M | water |
| nalidixic acid | 1/2 volume hot water | water |
| nitrofurantoin | 5 ml DMSO | water |
| tetracycline | water | water |
| norfloxacin | 1/2 volume water, minimal 0.1 N NaOH | water |

The sulfamethoxazole/trimethoprim stock solution was prepared by combining the individual sulfamethoxazole and trimethoprim stock solutions in a respective 3.71/1.95 ml ratio. All antibiotic stock solutions, except nitrofurantoin, were stored on ice until use. Nitrofurantoin may be stored at room temperature.

Example 4: Preparation of a negative growth control

A negative growth control was prepared to which no antibiotic was added. The negative growth control consisted of nutrient medium containing sodium azide (2.0 mg/ml; 0.2% solution) and ethylenediaminetetraacetic acid (EDTA, 0.2 millimolar), which acts to enhance the growth inhibiting action of sodium azide.

Because sodium azide is a bacterial growth inhibitor there should be no bacterial growth in this medium over the three hour incubation period. Therefore, any color development in this tube indicates too high of a bacterial concentration in the panel, i.e., bacterial overload. Or in this case of urine inocula, any color development in this tube indicates the presence of substances in the urine capable of reducing the indicator independent of the bacteria. As a result, purple color development in the sodium azide-EDTA tube invalidates the test.

Example 5: Preparation of MTT/phenazine methosulfate

The combination of 3-(4,5-dimethylthiazol-2-yl)-2,5-diphenyltetrazolium bromide (MTT) and phenazine methosulfate (PMS) was found to be the most suitable reagent system for detecting bacterial growth. Optimum concentrations of MTT and PMS were 0.2 millimolar each. Stock solutions (0.02 molar each) were prepared in DMSO and mixed at a 1:1 ratio.

The concentrations of MTT and PMS were tested at concentrations of 0.4 to less than 0.01 millimolar. At concentrations of 0.1 millimolar and less, color development (purple) required more time than did the 0.2 millimolar concentration. And, at concentrations of 0.4 millimolar, the color development included the production of more insoluble material with less brilliant formazan color. The concentration of PMS was kept to less than or equal to the concentration of MTT, otherwise intermediate reduced PMS (green) interfered with formazan production.

Example 6: Test procedure

For each clinical isolate or clinical urine to be tested, a set of sterile culture tubes was prepared. The set included a media control tube to show any color development in the absence of urine or bacterial inocula, a positive culture control without antibiotic added, a sodium azide-EDTA negative growth control tube, and a panel of nine different antibiotic tubes.

All of the tubes contained dextrose-supplemented Mueller-Hinton broth (0.5 ml), and the stock solutions of antibiotics were added to the nine antibiotic sensitivity testing tubes as shown in Table 3.

TABLE 3

| ANTIBIOTIC | ALIQUOT ADDITION ($\mu$l) | FINAL TUBE CONCENTRATION ($\mu$G/ml) |
|---|---|---|
| ampicillin | 16.13 | 16 |
| sulfamethoxazole/trimethoprim | 11.60 | 76/4 |
| sulfamethoxazole | 26.32 | 256 |
| trimethoprim | 7.93 | 8 |
| cephalothin | 16.13 | 16 |
| nalidixic acid | 16.13 | 16 |
| nitrofurantoin | 10.20 | 20 |
| tetracycline | 7.93 | 8 |
| norfloxacin | 10.20 | 8 |

The microorganism suspensions of Example 2 (10 $\mu$l) were added to each of the culture tubes in the test set as described in Example 6. Urine sample volumes ranged from 5 to 100 $\mu$l. For urine tests, the concentrations of ampicillin, cephalothin, and nalidixic acid were doubled to 32 $\mu$g/ml.

For purposes of the examples, the amount of urine sample to be used was determined either by Gram staining or control tube detection. In the Gram stain method one drop of uncentrifuged urine is placed on a slide to dry. The slide is fixed and Gram stained. The presence of one or more bacterial cells per oil immersion field correlates with a greater than 100,000 cell per milliliter concentration. At this concentration a urine inoculation volume of 100 $\mu$l is preferred. In control tube detection, positive control tubes as described in this invention are inoculated with 10 $\mu$l and 100 $\mu$l amounts of urine specimen into separate tubes. The tubes are incubated for three hours and reagents are added. If both the low and high inoculum tubes are positive, this indicates a high range of bacteria, and the low inoculum volume should be used in each tube of the actual antibiotic test panel. If only the higher inoculum control tube is positive, this indicates a low range of bacteria, and the higher inoculum volume should be used in each tube of the antibiotic panel.

All tubes were incubated for three hours at 37° C. Within 15 minutes of the three hour incubation time, the MTT/phenazine methosulfate preparation of Example 5 (10 to 20 $\mu$ls) was added to each tube. And, ten microliters of a 10% solution of Tween-80 in sterile water (1 ml/10 mls) were added to each tube.

Interpretation of results - Color Development

Bacterial growth was determined for the clinical urine inocula and bacterial isolates at the end of a three hour incubation by the addition of indicator/electron carrier (MTT/PMS) to each sample. Tubes with active bacterial metabolism turned purple, and those without active bacterial metabolism remained comparable to the negative control. Within 15 minutes of the addition of MTT/PMS, all tubes were graded either as resistant (purple) or sensitive (no purple). A range of intermediate color development may occur in some samples. Based on a 15 minute reading, the sample in question must have displayed some degree of purple to be graded antibiotic-resistant. Slight darkening (green) was graded antibiotic-sensitive.

The positive growth control tube for each known microorganism or positive urine should turn purple. If it does not turn purple, there is a problem in the system and any other color development in the set is invalid.

The test controls of *E. coli* ATCC #25922 should develop a similar pattern of resistance to the antibiotic panel each time it is run. If this does not occur, it may indicate a problem in the system, but does not necessarily invalidate the test.

Example 7: Correlation summary of clinical urine isolates

A bacterial isolate from each of the clinical urines was separately tested with a standard antibiotic resistance test. The latter test panel of antibiotics included ampicillin, sulamethoxazole/trimethoprim, cephalothin, nalidixic acid, nitrofurantoin, and tetracycline. Resistance and sensitivity scores were compared for the major classes of bacterial infections for these antibiotics. The results of the correlations between the present invention and standard antibiotic-susceptibility tests appear in Table 4. The present invention produced a greater than 90% agreement with the standard tests for all six bacterial classes.

TABLE 4

| BACTERIAL STRAIN | NUMBER OF CORRELATIONS | ERRORS | % FIT |
|---|---|---|---|
| *Escherichia coli* | 132 | 2 | 98.5 |
| *Proteus* | 88 | 5 | 94.3 |
| *Klebsiella* | 89 | 4 | 95.5 |
| *Enterobacter* | 52 | 4 | 92.3 |
| *Staphlococcus* | 69 | 3 | 95.7 |
| *Streptococcus* | 75 | 6 | 92.0 |

Example 8: Tube prototype clinical correlations

The results of the present invention for 107 urine specimens were also compared with the results of a standard clinical laboratory work-up (including plating of urine aliquots for bacterial growth) for both specificity and sensitivity. The test of specificity in the predictive absence of clinically important bacteria (i.e., the lack of color in the positive growth control) was 100% in the 38 negative urines. The test of sensitivity in detecting significant bacteria in urine (i.e., color in the positive growth control) matched the standard tests in 63 of 66 cases (95.5%). Three of the 107 samples were excluded from the correlations because they contained *Pseudomonas*, a slow growing bacteria, which requires incubation times longer than three hours and longer indicator reactions to produce color development

The antibiotic sensitivities of the urine inoculums having color in the positive growth control were compared to standard tests of clinical isolates taken from those urines in a manner similar to Table 4 for the antibiotics listed. Agreement of the results of the present invention with the standard isolate test was 90% or greater as shown in Table 5.

TABLE 5

| | ANTIBIOTIC | ANTIBIOTIC SENSITIVITY |
|---|---|---|
| | tetracycline | 93% |
| | furadantin | 98% |
| | sulfamethoxazole/trimethoprim | 95% |
| | nalidixic acid | 90% |
| | cephalothin | 95% |
| | ampicillin | 93% |

**Claims**

1. A method for determining the antibiotic resistance of a microorganism in a test sample, comprising the steps of:

a. adding the test sample, suspected of containing a microorganism, to a medium comprising a nutrient and an antibiotic which may be capable of interfering with the metabolism of the microorganism;

b. incubating said medium;

c. adding an indicator, capable of undergoing a color change in the presence of a microorganism, without substantially interfering with the metabolism of the microorganism, and an electron carrier, capable of facilitating the production of a color change of said indicator by the microorganism; and

d. detecting any color change produced by an antibiotic-resistant microorganism present in the test sample.

2. A method for determining the presence of a microorganism in a test sample, comprising the steps of:

a. adding the test sample, suspected of containing a microorganism, to a nutrient medium;

b. incubating said medium;

c. adding an indicator, capable of undergoing a color change in the presence of a microorganism without substantially interfering with the metabolism of the microorganism, and an electron carrier, capable of facilitating the production of a color change of said indicator by the microorganism; and

d. detecting any color change produced by a microorganism present in the test sample.

3. A kit for determining the antibiotic resistance of a microorganism, comprising:

(1) a nutrient;

(2) an antibiotic, which may be capable of interfering with the metabolism of the microorganism;

(3) an indicator, capable of undergoing a color change in the presence of a microorganism without substantially interfering with the metabolism of the microorganism; and

(4) an electron carrier, capable of facilitating the production of a color change of said indicator by the microorganism.

4. A kit for detecting the presence of a microorganism in a test sample, comprising:

(1) a nutrient;

(2) an indicator, capable of undergoing a color change in the presence of a microorganism without substantially interfering with the metabolism of the microorganism; and

(3) an electron carrier, capable of facilitating the production of a color change of said indicator by the microorganism.

5. The method according to Claim 1 or 2 wherein said indicator is a tetrazolium compound.

6. The kit according to Claim 3 or 4 wherein said indicator is a tetrazolium compound.

7. The method according to Claim 5, wherein said tetrazolium compound comprises, 2,3,5-triphenyl-2H-tetrazolium chloride (red tetrazolium); 2,5-diphenyl-3-[alpha-naphthyl]-1-tetrazolium chloride (tetrazolium violet); 3-(4,5-dimethylthiazol-2-yl)-2,5-diphenyl-2H-tetrazolium bromide (MTT); 2-(4-iodophenyl)-3-(4-nitrophenyl)-5-phenyl tetrazolium chloride (INT); 3,3'-(4,4'-biphenylene)bis(2,5-diphenyl-2H-tetrazolium chloride) (neotetrazolium); 3,3'-(3,3-dimethoxy-4-4'-biphenylene)bis(2,5-diphenyl-2H-tetrazolium chloride) (blue tetrazolium); 3,3′-(3,3′-dimethoxy-4,4′-biphenylene)bis[2-(4-nitrophenyl)-5-phenyl-2H-tetrazolium chloride] (nitroblue tetrazolium); or 3,3′-(3,3′-dimethoxy-4-4′biphenylene)bis[2,5-bis(4-nitrophenyl)-2H-tetrazolium chloride] (tetranitroblue tetrazolium).

8. The kit according to Claim 6, wherein said tetrazolium compound comprises, 2,3,5-triphenyl-2H-tetrazolium chloride (red tetrazolium); 2,5-diphenyl-3-[alpha-naphthyl]-1-tetrazolium chloride (tetrazolium violet); 3-(4,5-dimethylthiazol-2-yl)-2,5-diphenyl-2H-tetrazolium bromide (MTT); 2-(4-iodophenyl)-3-(4-nitrophenyl)-5-phenyl tetrazolium chloride (INT); 3,3′-(4,4′-biphenylene)bis(2,5-diphenyl-2H-tetrazolium chloride) (neotetrazolium); 3,3′-(3,3-dimethoxy-4-4′-biphenylene)bis(2,5-diphenyl-2H-tetrazolium chloride) (blue tetrazolium); 3,3′-(3,3′-dimethoxy-4,4′-biphenylene)bis[2-(4-nitrophenyl)-5-phenyl-2H-tetrazolium chloride] (nitroblue tetrazolium); or 3,3′-(3,3′-dimethoxy-4-4′biphenylene)bis[2,5-bis(4-nitrophenyl)-2H-tetrazolium chloride] (tetranitroblue tetrazolium).

9. The method according to claim 1 or 2 wherein said electron carrier comprises, N-methylphenozonium methosulphate (PMS); N-ethylphenazonium ethosulfate (PES); or phenazine N-oxide PNO).

10. The kit according to claim 3 or 4 wherein said electron carrier comprises, N-methylphenozonium methosulphate (PMS); N-ethylphenazonium ethosulfate (PES); or phenazine N-oxide PNO).

11. The method according to claim 1 wherein said antibiotic comprises, ampicillin, sulfamethoxazole, trimethoprim, sulfamethoxazole/trimethoprim, cephalothin, nalidixic acid, nitrofurantoin, tetracycline, or norfloxacin.

12. The kit according to claim 3 wherein said antibiotic comprises, ampicillin, sulfamethoxazole, trimethoprim, sulfamethoxazole/trimethoprim, cephalothin, nalidixic acid, nitrofurantoin, tetracycline, or norfloxacin.

13. The method according to Claim 1 or 2 further comprising the addition of a nonionic detergent to said medium.

14. The kit according to Claim 3 or 4, further comprising a nonionic detergent.

15. The kit according to Claim 3 or 4, further comprising a negative control composition which contains a growth inhibitor.